(19) Europäisches Patentamt
European Patent Office
Office européen des brevets

(11) Publication number: **0 101 785**
**B1**

(12) **EUROPEAN PATENT SPECIFICATION**

(45) Date of publication of patent specification: **05.08.87**

(21) Application number: **83103455.8**

(22) Date of filing: **08.04.83**

(51) Int. Cl.[4]: **C 07 C 149/20,**
C 07 C 149/23,
C 07 D 209/48,
C 07 D 209/76, C 08 K 5/37,
C 07 C 109/087

(54) **Organic compounds that contain sulphur, and their use as additives in polymers.**

(30) Priority: **09.04.82 US 367107**
**21.10.82 US 435779**
**08.11.82 US 439865**
**21.10.82 US 435780**
**21.10.82 US 435544**
**08.11.82 US 439864**

(43) Date of publication of application:
**07.03.84 Bulletin 84/10**

(45) Publication of the grant of the patent:
**05.08.87 Bulletin 87/32**

(84) Designated Contracting States:
**AT BE CH DE FR GB IT LI LU NL SE**

(56) References cited:
**DE-A-2 648 970**
**DE-A-2 756 612**
**US-A-4 100 132**
**US-A-4 147 689**

**CHEMICAL ABSTRACTS, vol. 80, no. 21, 27th May 1974, Columbus, Ohio, USA; W. TAKSHI "Polyolefin compositions", page 43, column 1, abstract no. 121888f**

(73) Proprietor: **CANUSA COATING SYSTEMS LIMITED**
**25 Bethridge Road**
**Rexdale Ontario M9W 1M7 (CA)**

(72) Inventor: **Hansen, Ralph Holm**
**7 Brooks Hill**
**Lincoln Massachusetts (US)**

(74) Representative: **Schwabe, Hans-Georg, Dipl.-Ing. et al**
**Patentanwälte Schwabe, Sandmair, Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 101 785 B1

Courier Press, Leamington Spa, England.

**Description**

Brief Statement of the invention

This invention is directed to a compound of the formula (I)

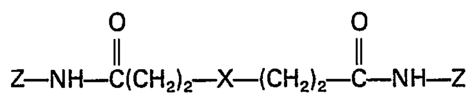

(I)

where Z is a group of the formula III

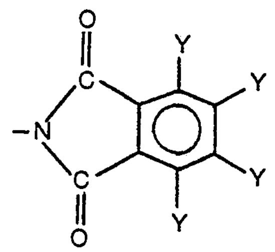

(II)

and X is S or S—S
Y is H, Cl or Br and at least one Y is Cl or Br or Z is a group of the formula III

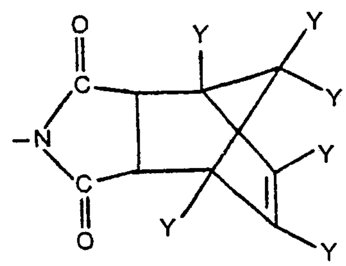

(III)

where X is S or S—S and Y is Cl or Br.

While it would be expected that the compound (I) would have nil antioxidant properties at elevated temperatures in a polymer, unexpectedly the compound of formula (I) appears to have antioxidant properties comparable to compounds which would be considered to be primary antioxidants. The compound will also, when used in combination with one or more primary antioxidants substantially and unexpectedly increase the useful life of the polymer at elevated temperatures without requiring the high loading of the primary antioxidants (also referred to as chain-breaking antioxidants).

While it may be possible to use additional antioxidants, to achieve the increased polymer life at elevated temperatures, primary antioxidants commonly used tend to diffuse out the polymer at high concentrations and form undesirable coating (termed blooming) on the surface thereof. In addition, the loss of primary antioxidant will over a period of time permit oxidation to take place at an accelerated rate and thereby significantly decrease the mechanical and electrical properties of the polymer thus shortening its lifetime. The commonly used antioxidant synergist (synergists are sometimes referred to as secondary antioxidants, and also referred to as peroxide-decomposing antioxidants; DLTDP (dilauryl thiodipropionate) also is known to bloom.

The present invention is useful in polymers used for electrical insulation, in heat-shrinkable tubing and other parts, e.g. end caps of polyethylene and used for electrical purposes, as well as in other plastic (polymer) parts used as utensils or as parts of the tubs of washing machines to prevent them from becoming brittle due to loss of antioxidant (because of soapy water causing the antioxidants commonly used to leach out of the plastic).

The compound of formula (I) is particularly useful in heat-recoverable (heat-shrinkable) articles of manufacture such as tubing, end caps and other hollow articles to which heat is applied to cause shrinkage because the lack of blooming permits coating with adhesives which may contain metal particles.

Polymers in which the compound of formula (I) are useful in this invention include all thermoplastics and thermo-hardening (thermosetting) plastics in which antioxidants are employed. Suitable plastics may include polyolefins such polyethylene (high and low density), polypropylene, polybutylene, substituted polyolefins such as halogenated olefin polymers and copolymers of same and silane-grafted polyethylenes, e.g. grafted using a silane such as vinyl trimethoxy silane as the grafting agent (see U.S. Patent No. 3,086,242).

The compound of formula (I) would also be useful with any polymer whose useful properties are adversely affected by oxidative degradation such as esters, amides (e.g. nylon), phenolics, acrylics, rubber, urethanes, vinyls, styrenes (e.g. ABS), and others used in the plastics industry. See the Text PLASTICS IN

THE MODERN WORLD by E.G. Couzens and V.E. Yarsly (C) 1968, published by Pelican Books, Inc., Maryland U.S.A., for other polymers used in industry and useful in this invention.

Prior art patents showing heat-recoverable plastics and articles include U.S. Patents 4,048,129, 4,016,356, 3,982,546 and 3,959,052. It should be understood that heat-recoverable articles are meant to include those that are treated by irridation or chemically treated to product such articles.

Examples of primary antioxidants useful in a polymer with the compound of formula (I) include:

ANTIOXIDANTS

| Commercial Name | Chemical Name |
|---|---|
| Irganox 1010 | tetrakis[methylene-3(3', 5'-di-tert-butyl-4'-hydroxyphenyl)propionate]methane |
| Santonox R | 4,4'-thiobis[3-methyl-6-tert-butylphenol) |
| Irganox 1024 | N,N'-bis(3,5-di-tert-butyl-4-hydroxyhydrocinnamoyl)hydrazine |
| Cyanox 1729 | Bis(4-tert-butyl-3 hydroxy-2,6-dimethylbenyzl)dithiolterephthalate |
| Ethyl 330 | 1,3,5,Trimethyl-2,4,6-tris[3,5 di-tert-Butyl-4 hydroxy benzyl]-benzene |
| Agerite White | di-β-naphthyl-p-phenylene-diamine |
| Irganox 1035 | thiodiethylene bis (3,5-di-tert-butyl-4-hydroxy) hydrocinnamate, |

Other suitable commercial antioxidants include Good-Rite 3114, Plastanox 2246, Naugard 445, Naugard XL-1, Irganox 1093, Irganox 1076, Topanol CA, and Irganox 565. Other antioxidants (normally termed primary antioxidants) in the art may be found in the text ANTIOXIDANTS, RECENT DEVELOPMENTS, CHEMICAL TECHNOLOGY REVIEW NO. 127, by M. William Ranney, Noyes Data Corporation (C) 1979, Library of Congress, Catalog No. 79-84425.

In using this invention to form polymeric articles, while the compound of formula (I) acts as a synergist, it is preferred that an amount of the compound of formula (I) to the amount of antioxidant is in the ratio of 1 : 10 to 10 : 1 with the total weight of antioxidant and the compound of formula (I) being within the range of .05 to 10% based on the weight of the polymer (resin).

The compound of formula (I) provides the sulfur for synergism with primary (strong) antioxidants (see list of antioxidants on previous page) and also includes
the hydrazone group

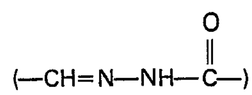

which is normally considered only as a mild antioxidant but a powerful copper (metal) deactivator. Accordingly, the compound of formula (I) could be very useful in polymers which contact copper, e.g. insulation for copperwire used in the telephone and power industry.

The compound of formula (I) is substantially insoluble in polymers (does not migrate) and is not volatile (substantially no weight loss after 13 hours at 200°C with a stream of oxygen flowing over it).

In using the compound of formula (I) by itself in a polymer, the concentration used should preferably be between .05 to 10% of the weight of the polymer depending upon environmental conditions to be met when used solely as an antioxidant. When used also as a flame retardant the total weight of the compound of formula (I) should be within the range of 0,05 to 150% of the weight of the polymer and is generally 50 to 100%.

In this invention, the preferred primary antioxidants are those characterized in the art as hindered phenolics or aromatic amines.

The compound of formula I where Y is Cl or Br and in addition to the antioxidant properties, also have particularly useful flame retardant properties and may be used primarily as flame retardants in polymer compositions.

Preferred compounds of the formula (I) include the compounds of formula (IC)

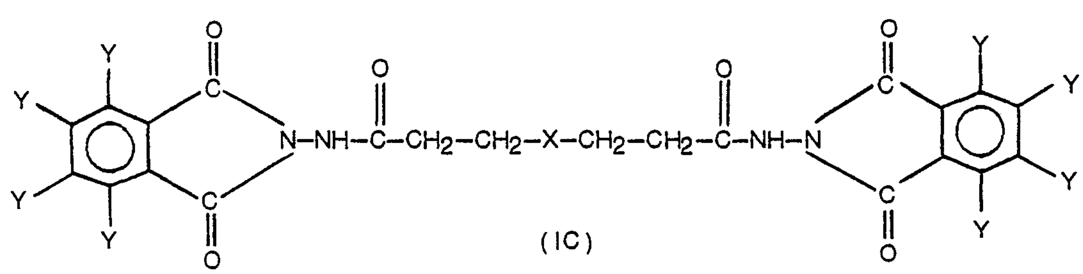

(IC)

where Y is H, Cl or Br and X is S or S—S;
and the compound of the formua (IE)

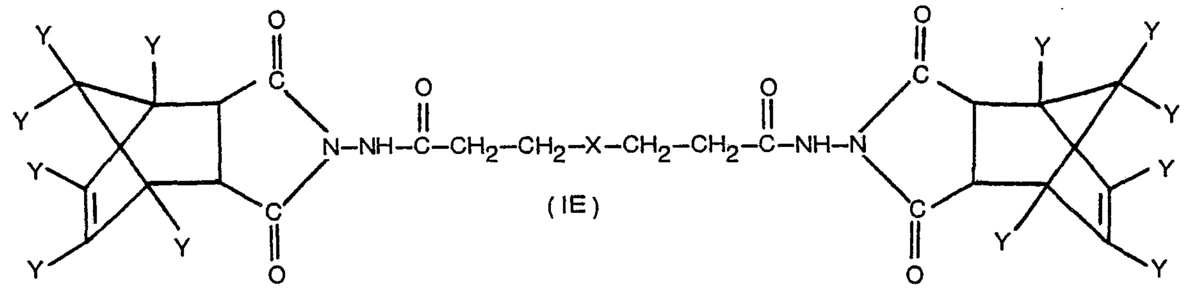

(IE)

where Y is defined as above.

The following examples are illustrative of the practice of the invention and are not intended for purposes of limitation. All parts are by weight and all temperatures are in centigrade.

Example 1

Preparation of the compound of formula (IC)
Where X is S and each Y is Br.

The compound of this example may be prepared in two steps as follows:

Sixty ml. of water is heated to about 60°C and 10 grams of dimethyl thiodipropionate

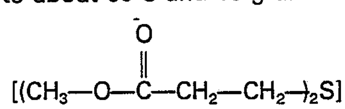

are added with stirring.

To this two-phase composition is added 7.3 grams of hydrazine hydrate $[NH_2NH_2]H_2O$ (50% excess) while stirring is continued. In about 5 minutes the suspension clarifies and reaction appears to be complete. Stirring is continued for additional 25 minutes and the solution is cooled to about 5°C (crystals form at about 35 to 40°C), filtered and dried. The yield is about 7.5 grams of thiodipropionic acid dihydrazide, m.p. 154°C (Perkin-Elmer DSC-2 calorimeter at a heating rate of 10°/minute). The yield may be increased by re-using the mother liquor in place of the water, by concentrating the mother liquor, or by diluting with a poor solvent for the dihydrazide such as methanol.

The thiodipropionic acid dihydrazide

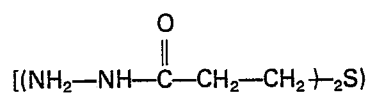

is used without further purification. To 300ml. of water at room temperature is added 10 grams of powered tetrabromophthalic anhydride and 10 grams of the thiopropionic dihydrozide. The mixture is stirred at room temperature for 1 hour and then the temperature is raised to approximately 100°C over a two-hour period. The solid is filtered, washed with water and dried. A nearly quantitative yield (19 grams) compound of formula (IC) is obtained.

This ditetrabromo compound (where each Y is Br and X is S) has a small endotherm at about 165°C (Perkin-Elmer DSC-2 calorimeter at a heating rate of 10°C/minute). It is substantially white and is insoluble in boiling water.

Example 2

Using the compound of example 1 a number of compositions are prepared by mixing the proportions of ingredients (percent by weight shown) into a polymer comprising 9% vinyl acetate — 91% ethylene copolymer (commercially known as U.S. Industrial Chemicals UE 635) on a heated, two-roll mill, molding into a sheet approximately 75 mils as shown below:

i) 0,5 part by weight of Naugard XL-I [2,2′-oxamidobis ethyl 3 (3,5-ditert-butyl-4-hydroxyphenyl) propionate] and 3 parts by weight compound of example 1 and 100 parts by weight of the polymer;

ii) 3 parts by weight of the compound of example 1 and 100 parts by weight of the polymer; and

iii) 1 part by weight of the compound of example 1 and 100 parts by weight of the polymer.

Example 3

Preparation of the compound of formula (IC) where X equals S—S and each Y is Br.

The dibenzyl derivative of the dihydrazide of thiodipropionic acid (compound of formula (IC)) may be prepared in two steps as follows:

Sixty ml. of water is heated to about 60°C and 11.6 grams of dimethyl dithiodipropionate

O
||
$(CH_3—O—C—CH_2—CH_2—S—)_2$

are added with stirring.

To this two-phase composition is added 7.4 grams of hydrazine hydrate $[NH_2NH_2]H_2O$ (a 50% excess) while stirring is continued. In about 5 minutes the suspension clarifies and reaction appears to be complete. Stirring is continued for an additional 25 minutes and the solution is cooled to about 5°C (crystals form at about 35 to 40°C), filtered and dried. The yield is about 8.5 grams of dithiodipropionic acid dihydrazide (M.P.131°C). The yield may be increased by re-using the mother liquor in place of the water, by concentrating the mother liquor, or by diluting with a poor solvent for the dihydrazide such as methanol.

The dithiodipropionic acid hydrazide

O
||
$(NH_3—NH—N—CH_2—CH_2—S—)_2$

is used without further purification. To 300ml. of water at room temperature is added 10 grams of tetrabromophthalic anhydride and 11.6 grams of the dithiodipropionic dihydradizde, with stirring. After one hour the temperature is raised to approximately 100°C over a two-hour period. The solid is filtered, washed with water and dried.

A nearly quantitative yield (19.5 grams) of the compound of formula (IC) is obtained. It is substantially white and is insoluble in boiling water.

Example 4

Using the compound of example 3 a number of compositions are prepared by mixing the proportions of ingredients (percent by weight shown) into a polymer comprising 9% vinyl acetate — 91% ethylene copolymer (commercially known as U.S. Industrial Chemicals UE 635) on a heated, two-roll mill, molding into a sheet approximatley 75 mils thick as shown below:

i) 0.5 part by weight of Naugard XL-1 [2,2'-oxamidobis ethyl, 3 (3,5-di-tert-butyl-4-hydroxyphenyl) propionate] and 3 parts by weight compound of example 3 and 100 parts by weight of the polymer;

ii) 3 parts by weight of the compound of example 3 and 100 parts by weight of the polymer; and

iii) 1 part by weight of the compound of example 3 and 100 parts by weight of the polymer.

Examples of commercially available materials from which compounds of formula (IC) are preparable include 4-chlorophthalic acid, 4,5-dichlorophthalic acid, phthalic anhydride; and pyromellitic dianhydride.

Example 5

Preparation of a compound of formula (IE) where each Y is Cl and X is S.

The compound of formula (IE) is prepared in two steps as follows:

Sixty ml. of water is heated to about 60°C and 10 grams of dimethyl thiodipropionate

O
||
$[(CH_3—O—C—CH_2—CH_2 )—_2S)$

are added with stirring.

To this two-phase composition is added 7.3 grams of hydrazine hydrate $[NH_2NH_2]H_2O$ (a 50% excess) while stirring is continued. In about 5 minutes the suspension clarifies and reaction appears to be complete. Stirring is continued for an additional 25 minutes and the solution is cooled to about 5°C (crystals form at about 35 to 40°C), filtered and dried. The yield is about 7.5 grams of thiodipropionic acid dihydrazide, m.p.154°C (Perkin-Elmer DSC-2 calorimeter at a heating rate of 10°/minute). The yield may be increased by re-using the mother liquor in place of the water, by concentrating the mother liquor, or by diluting with a poor solvent for the dihydrazide such as methanol.

The thiodipropionic acid hydrazide

O
||
$[NH_2—NH—C—CH_2—CH_2 )—_2S)$

is used without further purification. To 300 ml of water at room temperature is added 8 grams of powdered 1,4,5,6,7,7-hexachloro-norbornene-2,3,-dicarboxylic anhydride (chlorendic anhydride) and 10 grams of the thiopropionic dihydrazide, with stirring. After approximately one hour the temperature is raised to 100°C over a two-hour period. The material is washed with water, filtered and dried.

A nearly quantitative yield (17 grams) of the compound of formula (IE) where each Y is Cl and X is S is obtained.

The material melts at about 280°C (Perkin-Elmer DSC-2 calorimeter at a heating rate of 10°C per minute). It is substantially white and is insoluble in boiling water.

Example 6

Using the compound of formula (IE) prepared in example 5 a number of compositions were prepared by mixing the proportions of ingredients (percent by weight shown) into a polymer comprising 9% vinyl acetate — 91% ethylene copolymer (commercially known as U.S. Industrial Chemicals UE 635) on a heated, two-roll mill, molding into a sheet approximatley 75 mils thick as shown below:

i) 0.5 part by weight of Naugard LX-1 [2,2'-oxamido bis ethyl, 3 (3,5-di-tert-butyl-4 hydroxyphenyl) propionate] and 3 parts by weight compound of formula (IE) and 100 parts by weight of the polymer;

ii) 3 parts by weight of the compound of formula (IE) and 100 parts by weight of the polymer; and

iii) 1 part by weight of the compound of formula (IE) and 100 parts by weight of the polymer; and

iv) 0,47 part by weight of the compound of formula (IE) and 100 parts by weight of an ethylene homopolymer (commercially known as U.S. Industrial Chemicals NA 254).

Example 7

Preparation of the compound of formula (IC)
where each Y is Cl and X is S—S, the compound of formula (IE) is prepared in two steps as follows:

Sixty ml. of water is heated to about 60°C and 11.6 grams of dimethyl dithiodipropionate

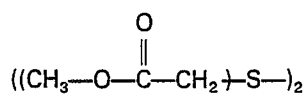

are added with stirring.

To this two-phase composition is added 7.3 grams of hydrazine hydrate $[NH_2NH_2]H_2O$ (a 50% excess) while stirring is continued. In about 5 minutes the suspension clarifies and reaction appears to be complete. Stirring is continued for an additional 25 minutes and the solution is cooled to about 5°C (crystals form at about 35 to 40°C), filtered and dried. The yield is about 7.5 grams of dithiodipropionic acid dihydrazide, m.p.131°C (Perkin-Elmer DSC-2 calorimeter at a heating rate of 10° per minute). The yield may be increased by re-using the mother liquor in place of the water, by concentrating the mother liquor, or by diluting with a poor solvent for the dihydrazide such as methanol.

11.6 gms of the dithiodipropionic acid dihydrazide

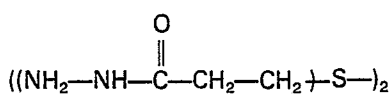

and 8 grams of powdered 1,4,5,6,7,7-hexachloro-norbornene-2,3,-dicarboxylic anhydride are used without further purification. To 300 ml. of water at room temperature is added the 11.6 grams and 8.0 grams of the above compounds, with stirring. After approximately one hour, the temperature is raised to 100°C over a two-hour period. The material is washed with water, filtered and dried.

A nearly quantitative yield (17 grams) of the compound of formula (IE) is obtained. This material is substantially white and is insoluble in boiling water and has a m.p. greater than 200°C.

Example 8

Using the compound of formula (IE) prepared in example 7 a number of compositions were prepared by mixing the proportions of ingredients (percent by weight shown) into a polymer comprising 9% vinyl acetate — 91% ethylene copolymer (commercially known as U.S. Industrial Chemicals UE 635) on a heated, two-roll mill, molding into a sheet approximatley 75 mils thick as shown below:

i) 0.5 part by weight of Naugard XL-1 [2,2'-oxamidobis ethyl, 3(3,5-di-tert-butyl-hydroxyphenyl)-propionate]; and 3 parts by weight of the compound (IE) of example 15 and 100 parts by weight of the polymer;

ii) 3 parts by weight of the compound of formula (IE) and 100 parts by weight of the polymer;

iii) 1 part by weight of the compound of formula (IE) and 100 parts by weight of the polymer; and

iv) 0.49 part by weight of the compound of formula (IE) and 100 parts by weight of an ethylene homopolymer (commercially known as U.S. Industrial Chemicals NA 254).

An example of a non-halogenated aliphatic carboxylic anhydride which can be used to prepare a non-flame retardant antioxidant of type (IE) is cyclohexane dicarboxylic anhydride.

**Claims:**

1. A compound of the formula I

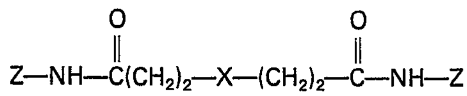

(I)

where Z is a group of the formula II

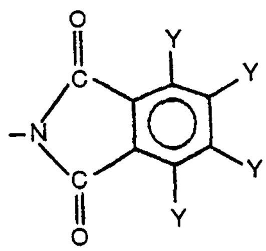

(II)

and X is S or S—S
Y is H, Cl or Br and at least one Y is Cl or Br or Z is a group of the formula III

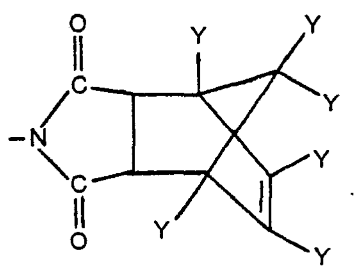

(III)

X is S or S—S
Y is Cl or Br

2. The compound of claim 1 in which Z is of the formula II and X is S and each Y is Cl.
3. The compound of claim 1 in which Z is of the formula II and X is S and each Y is Br.
4. The compound of claim 1 in which Z is of the formula III and X is S and each Y is Cl.
5. The compound of claim 1 in which Z is of the formula III and X is S—S and each Y is Cl.
6. The compound of claim 1 in which Z is of the formula III and each Y is Br.
7. The compound of claim 1 to 6 in a polymer.

**Patentansprüche**

1. Verbindung der Formel I

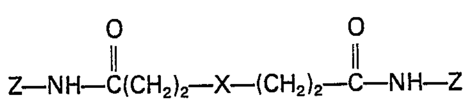

(I)

worin Z eine Gruppe der Formel II bedeutet

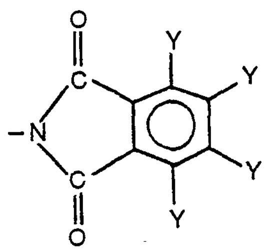

(II)

und X S oder S—S bedeutet,
Y H, Cl oder Br bedeutet und mindestens ein Y Cl oder Br bedeutet, oder worin Z eine Gruppe der Formel III bedeutet

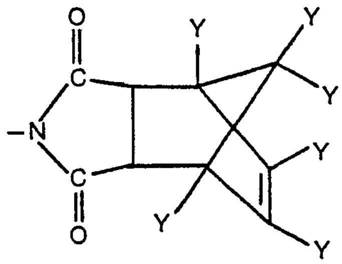

(III)

worin X S oder S—S bedeutet, und Y Cl oder Br bedeutet.

2. Verbindung nach Anspruch 1, worin Z die Bedeutung der Formel II aufweist und X S bedeutet und jedes Y Cl bedeutet.

3. Verbindung nach Anspruch 1, worin Z die Bedeutung der Formel II aufweist und X S und jedes Y Br bedeutet.

4. Verbindung nach Anspruch 1, worin Z die Bedeutung der Formel III aufweist und X S und jedes Y Cl bedeutet.

5. Verbindung nach Anspruch 1, worin Z die Bedeutung der Formel III aufweist und X S—S und jedes Y Cl bedeutet.

6. Verbindung nach Anspruch 1, worin Z die Bedeutung der Formel III aufweist und jedes Y Br bedeutet.

7. Verbindung nach Anspruch 1 bis 6 in einem Polymer.

**Revendications:**

1. Composé répondant à l formule I

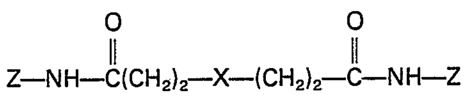

(I)

dans laquelle Z est un groupe répondant à la formule II

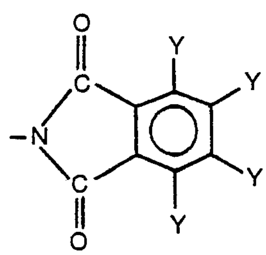

(II)

et X représente S ou S—S

Y représente H, Cl ou Br et au moins un Y représente Cl ou Br, ou Z représente un groupe répondant à la formule III

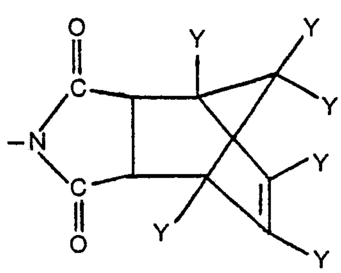

(III)

dans laquelle X représente S ou S—S et Y représente Cl ou Br.

2. Composé selon la revendication 1 dans lequel Z représente un groupe répondant à la formule II et X représente S et chaque Y représente Cl.

3. Composé selon la revendication 1 dans lequel Z est un groupe répondant à la formule II et X représente S et chaque Y représente Br.

4. Composé selon la revendication 1 dans lequel Z représente un groupe répondant à la formule III et X représente S et chaque Y représente Cl.

5. Composé selon la revendication 1 dans lequel Z est un groupe répondant à la formule III et X représente S—S et chaque Y représente Cl.

6. Composé selon la revendication 1 dans lequel Z est un groupe répondant à la formule III et chaque Y représente Br.

7. Composé selon la revendications 1 à 6 caractérisé en ce qu'il est contenu dans un polymère.